# EUROPÄISCHE PATENTSCHRIFT

(11) **EP 0 477 647 B1**
(45) Veröffentlichungstag und Bekanntmachung des Hinweises auf die Patenterteilung: **13.03.1996**
(21) Anmeldenummer: 91115239.5
(22) Anmeldetag: 10.09.1991
(51) Int. Cl.: C12P 19/26

(54) **Enzymatische De-Acylierung von Acyl-Aminosorbosen und dessen Verwendung bei der Herstellung von 1-Desoxynojirimicin**
Enzymatic deacylation of acyl-aminosorboses and its use for the production of 1-desoxynojirimicin
Désacylation enzymatique des acyl-aminosorboses et son utilisation pour la production d'1-desoxynojirimicine

(30) Priorität: 22.09.1990 DE 4030040
(43) Veröffentlichungstag der Anmeldung: 01.04.1992
(73) Patentinhaber: BAYER AG, D-51368 Leverkusen (DE)
(72) Erfinder: Schutt, Hermann, Dr., W-5600 Wuppertal (DE)

(56) Entgegenhaltungen:
- EP-A- 0 049 858
- EP-A- 0 240 868

## Beschreibung

Die vorliegende Erfindung betrifft ein verbessertes Verfahren zur Herstellung von 1-Desoxynojirimicin. 1-Desoxynojirimicin kann durch Alkylierung am Stickstoff-Atom zu verschiedenen Saccharase-Inhibitoren umgesetzt werden, die therapeutisch bei der Behandlung des Diabetes mellitus Anwendung finden (W. Puls, U. Keup, H.P. Krause, G. Thomas und F. Hoffmeister, Naturwissenschaften, 64 (1977) 536 ff).

1-Desoxynojirimicin wird durch ein kombiniertes chemisch-mikrobiologisches Verfahrenhergestellt (EP 49 858).

Eine wichtige Zwischenstufe bei der Herstellung von 1 Desoxynojirimicin ist die Aminosorbose, die jedoch instabil ist. Zur Abspaltung der N-Acetyl- und N-Formyl-Schutzgruppe müssen entweder extrem saure oder extrem alkalische Bedingungen gewählt werden. Oberhalb von pH 2 und unterhalb von pH 13 erfolgt praktisch keine Abspaltung der Acylgruppe. Bei den vorher genannten pH-Bedingungen werden jedoch beträchtliche Desoxynojirimicin-Verluste beobachtet. Diese resultieren einerseits aus Cyclisierungsreaktionen der Aminosorbose selbst zu z.B. hydroxytierten Pyridinen, andererseits bilden sich gefärbte, nicht näher bestimmte Nebenkomponenten. So wurde bei saurer Abspaltung der Acylgruppe nur eine Ausbeute von ca 27 % Desoxynojirimicin bei alkalischer Abspaltung eine Ausbeute von ca. 60 % Desoxynojirimicin beobachtet.

Überraschenderweise wurde nun gefunden, daß im Bereich von pH 8-9,5 die Abspaltung einer N-Phenacetyl-Schutzgruppe mittels des bekannten aus Escherichia coli isolierten Enyzms Penicillinacylase (Penicillin amido hydrolase (EC 3.5.1.11)) mit wesentlich besserer Ausbeute und Reinheit an 1-Desoxynojirimicin als mit chemischen Methoden erreicht werden kann.

Die genannte Reaktion wird durch die nachfolgenden Reaktionsgleichungen beschrieben:

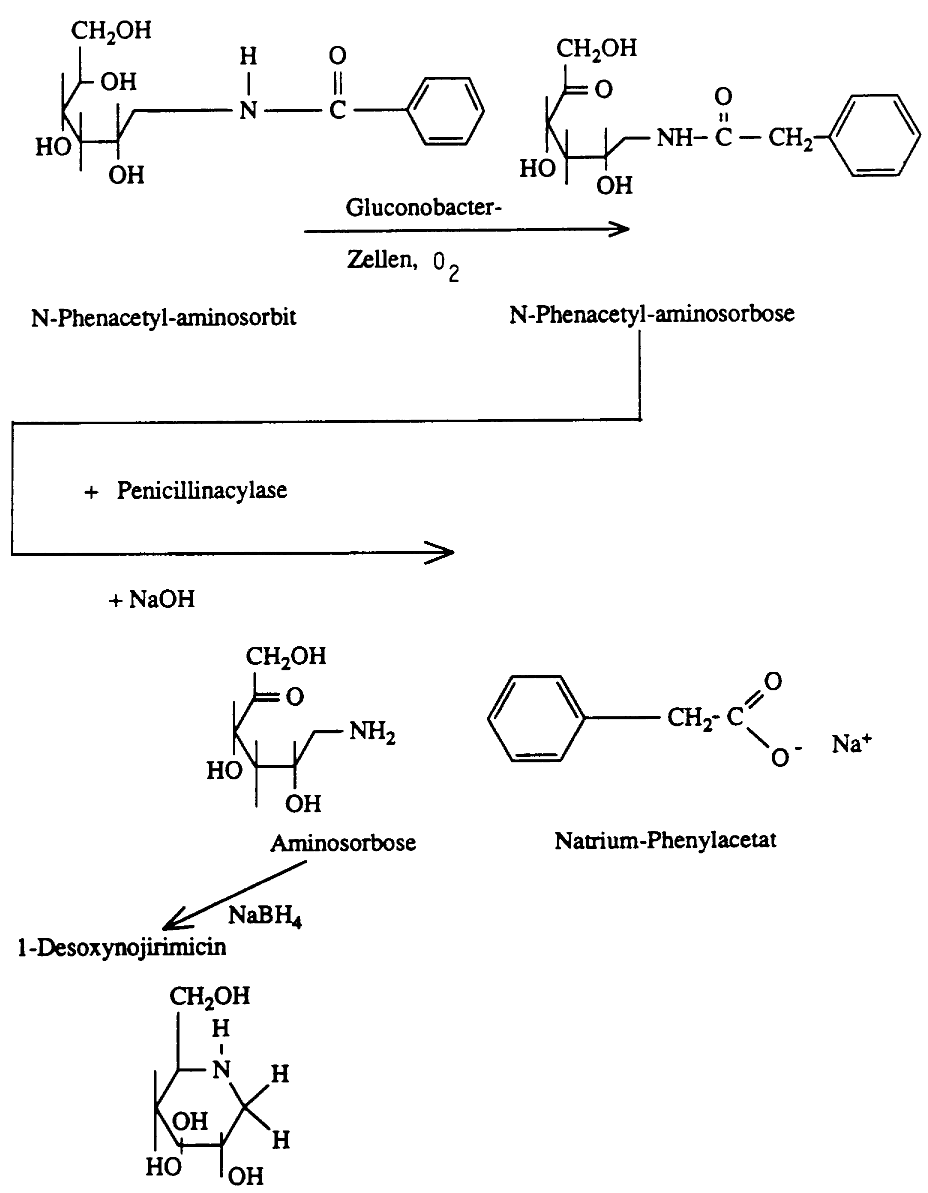

Bei pH 13 wird im Gegensatz zu pH 8-9 eine sehr schnelle Zersetzung von Aminosorbose beobachtet, die zum Verlust von 40-50% an 1-Desoxynojirimicin führt.

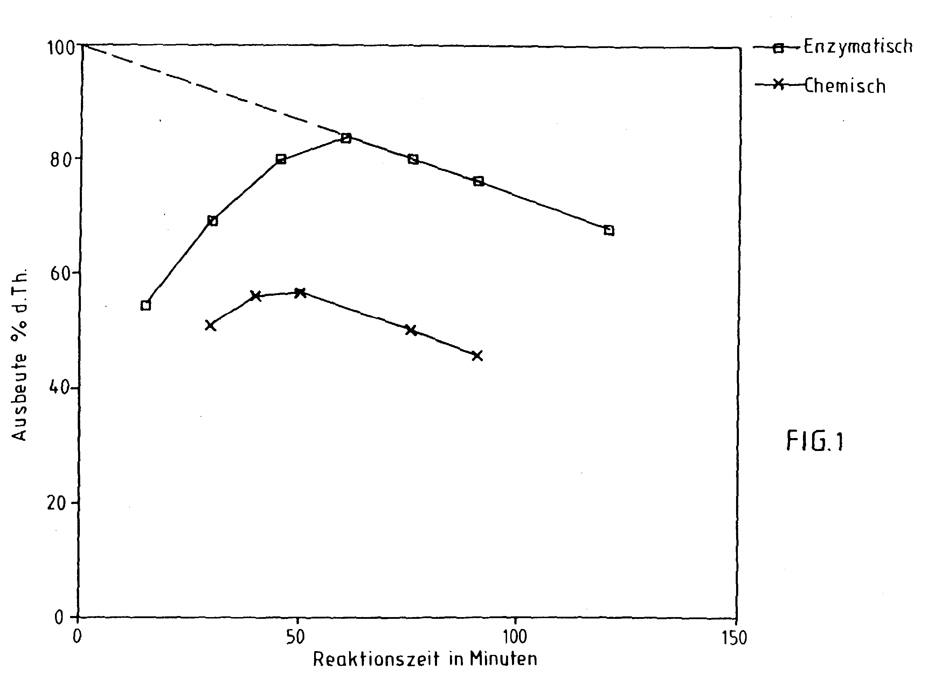

Bei der enzymatischen Abspaltung der N-Phenacetyl-Schutzgruppe mit Penicillinacylase jedoch fehlt diese schnelle Zersetzung, so das die Ausbeute an 1-Desoxynojirimicin gesteigert werden kann. Durch eine Steigerung der Enzymkonzentration und damit einer Verringerung der Abspaltzeit kann die Ausbeute noch weiter erhöhtwerden. (Abb. 1).

Penicillinacylase wurde bisher lediglich zur Abspaltung der N-Phenacetyl-Gruppe an Penicillin-, Cephalosporin-Grundkörpern, Aminosäuren und Peptiden beschrieben (J.G. Shewale, B.S. Deshphande, V.K. Sudhakaran und S.S. Ambedkar, Process Biochemistry, June 1990,97-103).

Die Abspaltung der Phenacetylgruppe mit Penicillinacylase von Aminozuckern ist neu und auch auf andere instabile N-Acyl-Aminopentosen und Aminohexosen anwendbar.

In den beschriebenen Verfahren können beispielhaft gereinigte freie und trägergebundene Penicillinacylase eingesetzt werden.

### Beispiel 1: Verwendung von löslicher Penicillinacylase

3 kg N-Phenacetyl-aminosorbit werden in 150 Litern Wasser gelöst und der pH-Wert der Lösung auf ca 4,5 eingestellt und bei diesem pH-Wert gehalten. Die Lösung wird auf 37°C erwärmt und ca 6 kg abgeschleuderte Bakterienzellen (Gluconobactesuboxydans) hinzugefügt und die Suspension für 4 Stunden mit Luft intensiv unter Rühren begast.

In diesem Zeitraum wird die N-Phenacetyl-aminosorbit quantitativ mikrobiologisch zu N-Phenacetyl-aminosorbose oxydiert. Die Gluconobacter-Zellen werden durch Zentrifugation vollständig aus der Suspension entfernt, der klare Überstand wird mit Natronlauge auf pH 9 eingestellt und 2,7 Millionen Einheiten Penicillinacylase (Isolierung des Enzyms und Definition der Einheit sind in der Literaturstelle: C. Kutzbach und E. Rauenbusch, Hoppe-Seyler's Z. Physiol.-Chem., 354 (1974) 45-53 beschrieben) zugefügt. Die Abspaltung der Phenylessigsäure wird mittels Hochdruckflüssigkeitschromatographie über die Bestimmung der freigesetzten Phenylessigsäure und/oder der Abnahme des N-Phenacetylaminosorbose verfolgt. Nach ca. 45-60 Minuten Reaktionszeit ist die Schutzgruppen-Abspaltung beendet und es werden 270 g Natriumborhydrid zugesetzt. Die Reduktion und die Cyclisierung zu 1-Desoxynojirimicin dauert 60 Minuten. Danach wird der Überschuß an Natriumborhydrid durch Zusatz von Aceton oder HCl zerstört.

Die weitere Aufarbeitung zum reinem 1-Desoxynojirimicin erfolgt durch Chromatographie über Ionenaustauscher und anschließende Kristallisation wie in EP 49 859 sowie in der Literaturstelle: Y. Ezure, S. Maruo, K. Miyazaki und M. Kawamata, Agric. Biol. Chem., 49 (1985) 1119-1125 beschrieben.

Nach Kristallisation werden ca. 1,3 kg 1-Desoxynojirimicin (ca 80 % molare Ausbeute) mit einer Reinheit von HPLC > 95 % erhalten.

### Beispiel 2: Verwendung trägergebundener Penicillinacylase

Die Durchführung erfolgt wie in Beispiel 1 beschrieben. Zur Abspaltung der N-Phenacetylgruppe werden 5,0 Millionen Einheiten Penicillinacylase-Harz hergestellt nach DOS 2 215 687 zugefügt. Vor der Isolierung von 1-Desoxynojirimicin wird das Enzymharz abgefiltert und gewaschen.

Die 1-Desoxynojirimicin-Ausbeute beträgt 1,4 kg (ca 83 % molare Ausbeute) mit einer Reinheit im HPLC > 95 %.

### Hochdruckflüssigkeitschromatographie

- Säule:: LiChrospher RP® 18,5 µm (Fa. Merck, Darmstadt); 250 mm * 4 mm
Laufmittel:
A: 0,05 M Phosphat pH 6,5
B: 90 % Acetonitril: 10 % Wasser
Gradient:

| Zeit inMin. | % A | % B |
|---|---|---|
| 0 | 100 | 0 |
| 2 | 100 | 0 |
| 10 | 0 | 100 |
| 11,5 | 0 | 100 |
| 11,6 | 100 | 0 |
| 16,5 | 100 | 0 |

Fluß: 1,5 ml/Min.
Temperatur: 25°C
Wellenlänge: 210 nm
Injektionsvolumen: 20 µL
Retentionszeiten in Minuten:

| | |
|---|---|
| N-Phenacetyl-Aminosorbit: | 7,1 |
| N-Phenacetyl-Aminosorbose: | 8,7 |
| Phenylessigsäure: | 4,7 |
| Desoxynojirimicin: | 0,9 |

## Patentansprüche

1. Verfahren zur Herstellung von 1-Desoxynojirimicin aus Acylaminosorbosen dadurch gekennzeichnet, daß die Deacylierung enzymatisch durch Penicillinacylase bei einem pH-Wert von 8 - 9,5 erfolgt.

## Claims

1. Process for the preparation of 1-desoxynojirimycin from acylaminosorboses, characterised in that the deacylation is carried out enzymatically by penicillin acylase at a pH of 8 - 9.5.

## Revendications

1. Procédé pour la préparation de la 1-désoxynojirimicine à partir d'acylaminosorboses, caractérisé en ce que la désacylation a lieu par voie enzymatique à l'aide de pénicillinacylase à une valeur de pH de 8-9,5.
